# EUROPEAN PATENT SPECIFICATION

(11) **EP 1 893 105 B1**
(45) Date of publication and mention of the grant of the patent: **26.11.2014**
(21) Application number: 06727096.7
(22) Date of filing: 11.05.2006
(51) Int. Cl.: A61B 17/22

(54) **BIOMECHANICAL PROBE**
BIOMECHANISCHE SONDE
SONDE BIOMECANIQUE

(30) Priority: 11.05.2005 GB 0509583
(43) Date of publication of application: 05.03.2008
(73) Proprietor: Likos Technologies Limited, Stratford upon Avon Warwickshire CV37 6JG (GB)
(72) Inventor: PEARCE, Gillian The University Of Wolvehampton, Wolverhampton WV1 1SB (GB); PERKINSON, Neil Donald, Darlaston, West Midlands WS10 9SQ (GB)
(74) Representative: Parnham, Kevin
(86) International application number: PCT/GB2006/001748
(87) International publication number: WO 2006/120464

(56) References cited:
- EP-A- 1 440 663
- WO-A-98/44982
- US-A- 4 767 404

## Description

The present invention relates to a probe and a device for use, in particular, in removing a blood clot from a part of the blood circulatory system. Also described is a method for removing a clot from a part of the blood circulatory system. The probe, device and method are particularly designed for use in the treatment of thrombo-embolic strokes.

The probe of the present invention is to be known as the Gwen Pearce (OP) Stroke Probe in acknowledgement of the lady who led to its conception.

Thrombo-embolic strokes account for about 85% of all strokes that occur and they occur when a clot forms or lodges in a part of the blood circulatory system leading to the brain, such as the middle cerebral artery. The formation of a clot stops blood supply to the brain through that part of the blood circulatory system and the effect is that the cells in the immediate vicinity of the clot, which area is called the Core, die within minutes. The cells outside the core but still in the area surrounding the blood clot, which area is called the Penumbral region, die within hours, typically up to 24 to 48 hours following the stroke.

At present the action taken in the case of a thrombo-embolic stroke is to simply observe the patient while administering an anti-coagulant to disperse the clot. A CT scan is carried out after 24 hours to determine the extent of the damage caused by the stroke. This course of action therefore may at least partially disperse the clot over a period of time but by the time the clot has been at least partially dispersed the maximum cell death may have already taken place. The patient is then often left with a degree of irreparable damage to the brain, which can result in various degrees of disability.

There are devices available for the removal of blood clots, in particular the use of inflatable balloon catheter devices has been known for many years.

When removing clots using inflatable balloon catheter devices the clot is generally located using fluoroscopy. The catheter is then inserted into the blood circulatory system and directed to the clot. The tip of the catheter is carefully moved through or beyond the centre of the clot and when the balloon has passed through the clot, the balloon is inflated. The catheter is withdrawn and the balloon ensures that the clot is pulled ahead of it.

Removal of clots using balloon catheter devices has many associated problems, for example there is the possibility of damage to the blood circulatory system; inflation pressures can create forces that can score the lining of part of the blood circulatory system, for example the artery, or dislodge plaque lodged on such a lining wall. It is also possible for the balloon to rupture leaving portions thereof in the bloodstream. Movement of the balloon can also displace the clot into other parts of the blood circulatory system rather then retrieve it.

Another type of device intended to remove clots comprises, for example, a catheter including a spiral helix designed to be rotated and pushed through the clot so that the helix screws into the clot, and then the catheter is pulled out of the blood circulatory system without rotation.

Catheters for removing materials from various body organs are also known and may have a net, or number of arms, which may be collapsed and inserted into an organ and past the material to be retrieved. The net is, or arms are, then unfolded and materials are removed from the organ.

The use of devices that are inflatable or expandable can lead to further damage as set out above by damage to the blood circulatory system wall being caused, plaque being dislodged and possible displacement of the clot.

It is also known to use catheters that break up clots and draw the broken up clot into the catheter by, for example, the movement of an impeller within the catheter body. Such catheters generally have a rotatable impeller within the catheter body. The rotatable impeller may assist in one or both of breaking up the clot and drawing the broken up clot out of the blood circulatory system into the catheter.

The use of devices with rotatable or movable parts increases the likelihood of damage to the walls of the blood circulatory system. Furthermore, as explained above, moving parts can lead to the clot becoming macerated or shredded giving rise to additional undesirable debris in the blood circulatory system. If this debris is not collected by the catheter it could circulate in the blood and lead to further, serious problems for the patient. In addition, owing to the presence of moving parts the catheter will generally need to be larger than a device with no moving parts and will be more complex and costly to make.

Therefore, there still remains a need for a device to allow retrieval of a clot from a blood circulatory system whilst limiting the possibility of additional damage to the patient. A device for removal of surgical debris with reduced clogging and with minimum trauma to tissue is disclosed in US-A-4767404. There is also a need to remove the clot as quickly as possible to reduce or eliminate cell death in the Penumbral region.

According to a first aspect the present invention provides a probe as claimed in claim 1 and dependent features in claims 2-16.

The phrase "blood circulatory system" is intended to include all parts of the body through which blood circulates, including arteries, veins and capillaries. Hereinafter, for simplicity, the phrase "blood vessel" will be understood to include all the parts of the blood circulatory system.

The probe may be generally cylindrical in configuration. Alternatively the probe may have a suitable polygonal external surface.

The formation of the channel in the probe forms a wall around the channel. The channel may be of constant diameter. Alternatively the aperture in the first end may be of greater diameter than the aperture in the second end. The channel between the second end and first end of the probe may be, or may have a portion, of increasing diameter as it extends from the second end to the first end. More preferably the channel has a portion of increasing diameter positioned close to the first end. It is most preferred that the diameter increases such that the wall of the channel is at an angle of 20 to 40°, preferably 30°, to the longitudinal axis of the channel. Therefore the channel flares out close to the first end to give a portion of increasing diameter, i.e. a frusto-conical portion.

The presence of a portion of increasing diameter is advantageous in assisting location and securing of the probe into means to connect it to a suction pump. The portion of increased diameter allows the second end of the probe to be pushed into the connection means until the portion of increased diameter at the first end of the probe prevents any further advancement of the probe into the connection means. The portion of increased diameter also provides a rounded end to the probe, which reduces damage to the blood vessel wall. The portion of increased diameter also assists in channelling and guiding the flow of blood and the blood clot into the probe.

The use of a probe that has no moving parts and is adapted in use to create a vortex when used with a suction pump allows the clot to be removed from the wall of the blood vessel with minimal damage to the blood vessel wall, the clot is then sucked into the probe thus preventing it from becoming displaced and causing further damage. It is thought that the use of a vortex allows the clot to be rolled from the wall of the blood vessel. The vortex is created by the shape and configuration of the probe and does not require any moving parts to function. Any other material dislodged during the procedure will also be sucked into the probe, again preventing further damage to the patient.

The probe may be provided with rifling on all or part of the wall of the channel. The rifling may be provided on the surface of the wall of any portion of the channel of increased diameter. Rifling helps to induce the vortex when used with a suction pump.

Alternatively the probe may be provided with one, two or more, preferably three, spirals of wire secured along at least part of their length to the surface of the wall of the channel. Preferably the spirals of wire are separate from each other. Preferably the spirals of wire are positioned equidistantly around the circumference of the channel most preferably at an angle of 30° to the longitudinal axis of the probe. The use of spirals of wire can be beneficial when the probe is very small and it may not be easy to accurately rifle the channel of the probe or to create a sufficient depth of rifling.

Preferably the wires are stainless steel or titanium and the spirals may be fused to the wall of the channel of the probe. Alternatively the spirals of wire may be formed in a single operation, for example they may be injection moulded with the probe. The spirals and probe are preferably made from the same material.

Alternatively the surface of the wall of the channel of the probe may be provided with one, two or more, preferably three, indented spirals along at least part of it's length. Preferably the spirals are separate from each other. Preferably the spirals are positioned equidistantly around the circumference of the channel most preferably at an angle of 30° to the longitudinal axis of the probe.

As a further alternative the probe may be provided with one, two or more, preferably three, spirals of suitable material secured along at least part of their length to the surface of the wall of the channel. Preferably the spirals of material are separate from each other. Preferably the spirals of material are positioned equidistantly around the circumference of the channel most preferably at an angle of 30° to the longitudinal axis of the probe. Preferably the material is titanium and the spirals may be adhered to the wall of the channel by an appropriate adhesive or alternatively fused to the wall of the channel of the probe, for example by use of induction or electrical current heating.

Alternatively, the spirals of material may be formed on the wall of the channel by three dimensional printing. The materials used for three dimensional printing are preferably materials compatible with the printing process and also with use in the human body, such as a suitable polymeric material. In this embodiment the probe may be manufactured from a sheet of suitable material on which the spirals can be formed by three dimensional printing before the probe is formed from the sheet material.

Where the probe is provided with rifling, with wire spirals or with spirals of material on all or part of the wall of the channel the rifling, wire spirals or spirals of material are preferably positioned at 20 to 35° and most preferably 30° to the longitudinal axis of the probe.

As a further alternative, or in addition to the presence of spirals or rifling, the probe may be provided with an elongate bar extending along the channel and provided with one, two or more spirals of wire secured around at least part of its length. Preferably the spirals of wire are separate from each other. Preferably the spirals of wire are positioned equidistantly around the circumference of the elongate bar most preferably at an angle of 30° to the longitudinal axis of the bar.

Alternatively the elongate bar may be a screw-threaded bar.

The elongate bar is preferably secured in the channel of the probe so that a flow path is created around the bar thus leading to the creation of a vortex when in use. In a most preferred embodiment the spirals of wire or the screw thread of the bar contact the wall of the channel and hold the bar in place either by a push fit or by a suitable fusing method such as welding. The elongate bar may alternatively be cast or moulded integrally with the probe.

Another alternative way to create the vortex is to introduce an irregularity into a straight flow of fluid, i.e. blood. This may be achieved by providing a bar across a part of the aperture in the first end of the probe. The bar preferably has a length of at least 40% of the diameter of the aperture and extends from one part of the edge of the aperture to another part of the edge of the aperture. Preferably the bar extends across the diameter of the aperture. The bar may be any suitable cross section, for example it may have a 'V' shaped profile, a square, semi-circular or triangular cross section or it may be a screw threaded bar.

This introduction of irregularity into a straight flow produces two opposing vortices.

In the probe any one means to create a vortex around the first end of the probe may be provided alone or in combination with any other means to create a vortex. The probe will have no moving parts.

The second end of the probe may include a collar extending from the second end around the channel; this collar may be sized and shaped to enter into male-female engagement with the connection means. The collar may include a locking means such as a screw thread or protrusions to correspond with a screw thread or apertures on the corresponding surface of the connection means. The provision of the collar can ensure that the probe tip and connection means are 'flush' which decreases the chance of damage to the patient and increases the strength of the connection.

Preferably the probe is releasably connected to a pump.

The probe must be made of a suitable material to allow it to be tracked when in the human body. For example the probe should ideally be visible to X-ray and angiogram machines. Alternatively the probe should be visible to magnetic resonance angiography.

The probe can be manufactured from any suitable material such as a plastics or flexible material, for example polypropylene, or a metal, for example stainless steel, titanium, aluminium or anodised aluminium. There is also the possibility of the probe being made from a plastics material or from a flexible material, such as polyether-polyamide co-polymer/polyether block amide (PEBAX), polypropylene or Teflon. The probe could also be made from a ceramic. Where the probe is not made from a metal it may be provided with a metal portion.

The metal portion allows the probe to be visible to X-rays during an angiogram procedure and therefore the metal portion may be provided by the presence of a metal strip or ring, for example a stainless steel band having a width of 1mm and a thickness of 0.35mm. The metal ring or strip preferably extends around all or part of the probe, more preferably around an exterior surface of the probe.

In an alternative embodiment the metal strip or ring may be provided extending around all or part of the collar extending from the second end of the probe around the channel, more preferably around an exterior surface of all or part of the collar.

In either of the aforementioned embodiments the strip or ring may be provided in a recessed slot or groove.

The metal portion may alternatively be provided by use of a metal containing, or metal loaded, plastics or polymeric material to form the probe.

The metal portion may be a lining extending along all or part of the channel through the probe, for example a stainless steel or titanium lining, to the channel extending between the first and second apertures of the probe. Such a lining would form the wall of the channel through the probe and would therefore bear any rifling or spirals present to form a vortex.

For use with magnetic resonance angiography the probe should be made from a non-magnetic material, for example aluminium.

The probe may be disposable or sterilisable.

The probe may be any suitable size depending on the blood vessel in which it is to be used. The probe may have a length of from 7 to 40mm, preferably from 10 to 25mm, more preferably from 13 to 20mm, most preferably the length of the probe is 15mm. The length of the probe helps establish a reliable vortex.

The diameter of the channel between the first and second apertures of the probe is preferably from 0.2 to 7mm, more preferably from 0.5 to 5.5mm. For example, for the middle cerebral artery the diameter of the channel may be 0.5 to 2.2mm, preferably 1.8mm; for a small artery such as the carotid artery the diameter of the channel may be 1.8 to 3mm, preferably 2.7mm and for a major artery such as the aorta the diameter of the channel may be 2 to 7mm, more preferably 3 to 7mm, most preferably 5mm. In this case the diameter of the channel does not consider any portion of increased diameter.

The thickness of the wall of the probe must be sufficient to give structural integrity to the probe and sufficient to avoid deformation when the probe is in use and suction is applied. The thickness of the wall of the probe is preferably up to 1mm, more preferably from 0.15 to 0.25mm.

The probe may be provided with one or more protrusions, or fins, extending from the outer surface thereof, these protrusions may support the walls of the blood vessel, particularly veins which have thin walls, when the probe is in use and allow the blood to continue to flow around the probe. The or each protrusion preferably extends along all or part of the length of the probe.

It is envisaged that the probe will be provided in a number of sizes for use in different parts of the body. Besides the extra large probe for use in a major artery, such as the aorta, there will be three general size ranges of probe.

It is envisaged that the large size of probe will generally have a length of from 20 to 30mm, preferably 25mm. The large size of probe will preferably have an outer diameter of from 2.5 to 3.5mm. The aperture in the first end of the largest size of probe will preferably be of greater diameter than the aperture in the second end. The channel between the second end and first end of the probe will preferably be, or have a portion, of increasing diameter as it extends from the second end to the first end. More preferably the channel will have a portion of increasing diameter positioned close to the first end. The channel should preferably have a length of at least 12mm between the second end and the portion of increasing diameter. It is most preferred that the diameter increases such that the channel wall is at an angle of 20 to 40°, preferably 30°, to the longitudinal axis of the channel. Therefore the channel flares out close to the second end to give a portion of increasing diameter, i.e. a frustoconical portion.

It is envisaged that a medium size of probe will generally have a length of from 15mm to 25mm, preferably 20mm. The medium size of probe will preferably have an outer diameter of from 0.8 to less than 2.5mm. The medium size of probe may be generally cylindrical in configuration.

It is envisaged that the smallest size of probe will generally have a length of from 10 to 20mm, preferably 15mm. The smallest size of probe will preferably have an outer diameter of less than 0.8mm. For the smallest size of probe the diameter of the channel between the first and second apertures of the probe is preferably from 0.2 to 0.6mm. The smallest size of probe may be generally cylindrical in configuration.

Any of the means discussed above for creation of a vortex can be used, alone or in combination, with the three sizes of probe. However, for the smallest size of probe it is envisaged that the provision of a bar across a part of the aperture in the first end of the probe or the use of the elongate bar as described above may be most useful whereas for the medium and large sizes of probe the use of spirals of wire, indented spirals or rifling may be more appropriate.

According to a second aspect the present invention provides a device for retrieving a blood clot from a blood vessel comprising a probe according to the first aspect of the invention and a suction pump, wherein the probe is connected to the suction pump by a connection means extending between the suction pump and the second end of the probe.

The second end of the probe may include a collar extending from the second end around the channel, this collar may be sized and shaped to enter into male-female engagement with the connection means. The collar may include a locking means such as a screw thread or protrusions to correspond with a screw thread or apertures on the corresponding surface of the connection means. The provision of the collar can mean that the probe and connection means are 'flush' which decreases the chance of damage to the patient and increases the strength of the connection. This can occurs when the collar forms either the male or the female part of the engagement between the connection means and probe.

The pump may be any suitable type of suction pump, for example a vacuum pump or a peristaltic pump. The suction pump may be provided with a filter.

The suction pump is preferably provided with an appropriate control means. The pressure applied by the suction pump is preferably controlled and monitored. The pressure may be adjusted or cut-off as a result of continuous monitoring of the pressure in use and any change in pressure. This prevents excessive amounts of blood from being removed from the patient. Up to approximately 180ml of blood can be safely removed from the patient but the present invention aims to remove no more than 40 to 60ml of blood from the patient.

The device is preferably provided with a collection vessel and a means to monitor suction of a blood clot into the collection vessel. On collection of a blood clot in the collection vessel the control means preferably decreases or cuts-off the pressure from the suction pump. The means to monitor suction of a blood clot into the collection vessel may be associated with the collection vessel. Alternatively the means to monitor suction of a blood clot into the collection vessel may comprise a flow meter associated with the connection means. The flow meter may be a magnetic flow meter.

The suction pressure applied by the suction pump must be sufficient to create a velocity in the blood flow that will lead to the formation of a vortex. Suitable pressures are 10 to 35mm Hg, more preferably 15 to 28mm Hg.

The use of a device according to the invention in the retrieval of a blood clot from a blood vessel comprising the steps of:
securing the connection means between the second end of the probe and the suction pump
inserting the probe into the blood vessel;
positioning the probe at a suitable distance from the blood clot;
applying suction through the probe from the suction pump to create a shear force in the form of a vortex at the first end of the probe to cause the blood clot to move from the blood vessel into the probe;
removing the probe from the blood vessel.

The probe is preferably positioned just far enough from the blood clot to allow the vortex to be created, a suitable distance is up to 5mm, preferably 3 to 5mm.

The preferred suction pressure applied by the pump, which is preferably a vacuum pump, to create a suitable velocity in the blood and therefore a vortex is 10 to 35mm Hg, more preferably 15 to 28mm Hg.

The blood vessel into which the probe is inserted is preferably an artery such as the femoral artery or carotid artery.

The insertion of devices, such as catheters, into the artery is known, for example in the clipping of aneurysms or in angiography, which is the location of abnormalities in the artery using dyes, and a similar technique would be used in the present invention to locate the clot and insert the probe.

The blood clot may remain in the probe whilst the probe is removed from the patient and this will lead to the probe being blocked and therefore the prevention of unnecessary loss of blood.

If the clot is sucked through the probe and into the collection vessel the means to monitor suction of a blood clot into the collection vessel would preferably cut-off the pump suction, again minimising unnecessary blood loss.

Preferably the method further comprises using an angiogram to determine whether a thrombo-embolic stroke has occurred before inserting the probe into the patient.

Preferably the position of the probe is tracked using X-rays during an angiogram procedure or magnetic resonance angiography.

The vortex created may be perpendicular to the longitudinal axis of the channel of the probe tip or alternatively may be parallel or co-axial with the longitudinal axis of the channel of the probe (modified Von Karman vortex).

The devices and method can be used to treat patients who have undergone a stroke, the treatment can be carried out quickly and without the need to wait for a CT scan.

It is envisaged that as soon as a stroke patient was seen they would be subjected to an angiogram to determine the nature of their stroke and if it was seen to be a thrombo-embolic stroke the device and method described could be used immediately to retrieve the clot before extensive damage was done to the patient. Angiograms can be carried out quickly and cheaply by lesser-trained medical staff therefore diagnosis could be carried out quickly and the patient could be treated in a time period of less than eight hours therefore minimising more extensive permanent damage to the brain by saving cells in the Penumbral region before they die off.

The devices of the present invention can also be used to retrieve blood clots from a patient before they begin to cause problems to the patient and therefore would help decrease the likelihood of strokes occurring.

The device of the present invention will now be described in further detail with reference to the drawings in which:
**Figure 1a** shows a cross section through a probe according to one embodiment of the present invention;
**Figure 1b** shows an end view of the probe of Figure 1a;
**Figure 2a** shows a cross section through a probe according to a second embodiment of the present invention;
**Figure 2b** shows an end view of the probe of Figure 2a;
**Figure 3a** shows a cross section through a probe according to a third embodiment of the present invention;
**Figure 3b** shows an end view of the probe of Figure 3a;
**Figure 4** shows an expanded view of a probe according to a fourth embodiment of the present invention; and
**Figure 5** is a schematic drawing of the device as a whole including a probe of any of Figures 1 to 4.

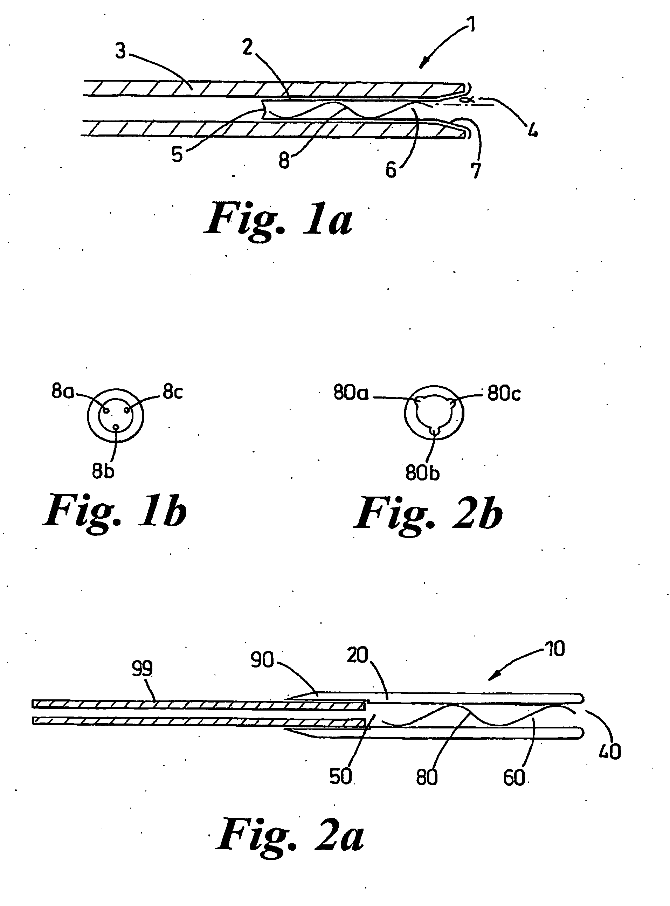

Figure 1a shows a probe 1 which is in the form of a stainless steel lining 2 inserted in a free end of a flexible tube 3, which is of suitable length and material to be connected to a suitable suction pump. The lining 2 has a first end 4 and a second end 5. A channel 6 passes through the probe from the second end 5 to the first end 4. The channel 6 is provided with a flared portion 7 as it extends from the second end 5 to the first end 4. The aperture that forms the end of the channel 6 at the first end 4 is of greater diameter than the aperture forming the end of the channel 6 at the second end 5.

The flared portion 7 preferably flares out at an angle α of 30° to the longitudinal axis of the channel 6.

The surface of the channel 6, between the second end 5 and the flared portion 7, is provided with means 8 to create a vortex, which comprises three spirals of wire 8a, 8b, 8c positioned thereon. The wires are spaced equidistantly round the circumference of the channel 6, as shown best in Figure 1b. The spirals of wire are preferably made of titanium and are fused to the surface of the channel 6. The spirals of wire create irregularity in the blood flow sufficient to give rise to the formation of a vortex.

The choice of the size of the probe to be used will be in the hands of a skilled operator, such as a surgeon and will depend on the site of the blood clot and its size.

The probe 1 shown is of a size and shape that could be used to retrieve a blood clot from the carotid artery and therefore has a length 20 to 25mm and an outer diameter of 2.5 to 3.5mm, including the flexible tubing 3. The diameter of channel 6 is 1.8 to 3.0mm and the distance from the first end 4 to the point in the channel 4 where the flared portion 7 begins is 4 to 5mm.

Figure 2a shows a probe 10, which is in the form of a stainless steel tubular member 20. The tube 20 has a first end 40 and a second end 50. A channel 60 passes through the probe from the second end 50 to the first end 40.

The surface of the channel 60 is provided with means 80 to create a vortex, which comprises three inscribed spirals 80a, 80b, 80c positioned therein. The spirals are spaced equidistantly round the circumference of the channel 60, as shown best in Figure 2b. The spirals are inscribed into the surface of the channel 60 during the manufactured process. The spirals create irregularity in the blood flow sufficient to give rise to the formation of a vortex.

The second end 50 of the probe 10 is provided with a collar 90 extending therefrom and extending around the circumference of the channel 60. The collar 90 is sized to be connected to a connecting means 99 to connect the probe 10 to a suction pump (not shown). The connecting means 99 is received within the collar 90 of the probe 10 by means of male female engagement and the collar 90 is shaped to be flush with the connecting means 99.

The choice of the size of the probe to be used will be in the hands of a skilled operator, such as a surgeon and will depend on the site of the blood clot and its size.

The probe 10 shown is of a size and shape that could be used to retrieve a blood clot from the middle cerebral artery and therefore has a length 18 to 20mm and an outer diameter of from 0.8 to less than 2.5mm. The diameter of channel 60 is 0.5 to 2.2mm.

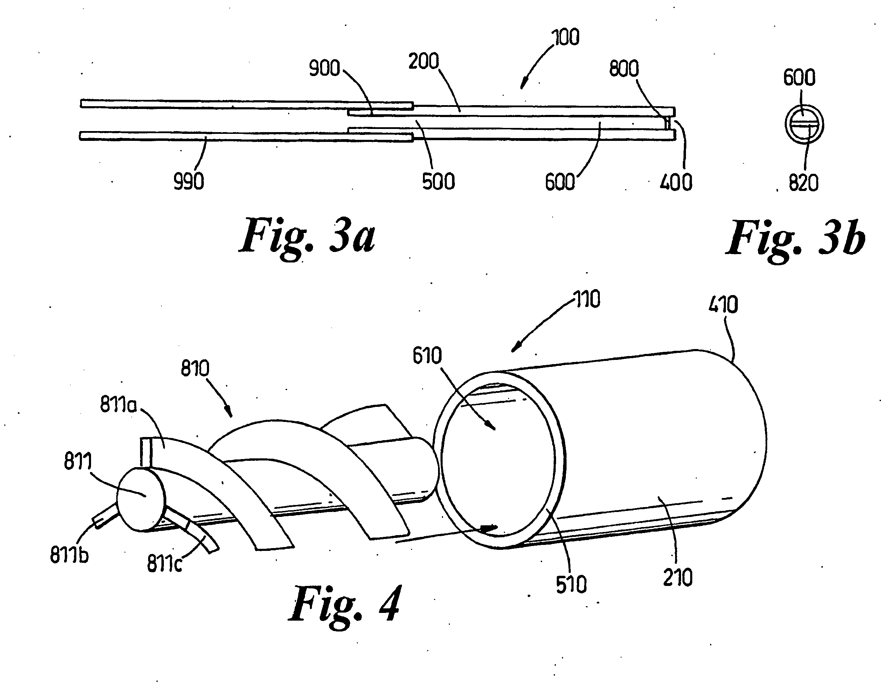

Figure 3a shows a probe 100, which is in the form of a stainless steel tubular member 200. The tube 200 has a first end 400 and a second end 500. A channel 600 passes through the probe from the second end 500 to the first end 400.

The channel 600 is provided with means 800 to create a vortex, which comprises an obstruction 820 extending across its diameter, as shown in Figure 3b. The obstruction 820 is a V-shaped bar which in use introduces an irregularity into a straight flow and creates two opposing vortices. The bar 820 is made of stainless steel and may be integral with or fused to the tubular member 200. In this design the clot is more likely to be sucked into the collection vessel than held in the probe as the probe is removed.

The second end 500 of the probe 100 is provided with a collar 900 extending therefrom and extending around the circumference of the channel 600. The collar 900 is sized to be connected to a connecting means 990 to connect the probe 100 to a suction pump (not shown). The collar 900 is received within the connecting means 990 by means of male female engagement and the collar 900 is shaped so that the outer surface of the probe 100 is flush with that of the connecting means 990.

The choice of the size of the probe to be used will be in the hands of a skilled operator, such as a surgeon and will depend on the site of the blood clot and its size.

The probe 100 shown is of a size and shape that could be used to retrieve a blood clot from near the apex of the middle cerebral artery and therefore has a length 18 to 20mm and an outer diameter of less than 0.8mm. The diameter of channel 600 is less than 0.6mm.

Figure 4 shows a probe 110, which is in the form of a stainless steel tubular member 210. The tube 210 has a first end 410 and a second end 510. A channel 610 passes through the tube from the second end 510 to the first end 410.

The channel 610 is provided with means 810 to create a vortex, which comprises an obstruction 810 extending longitudinally along the centre of channel 610. The obstruction 810 is an elongate bar 811 which has three spirals of wire 811a, 811b, 811c positioned thereon. The wires are spaced equidistantly round the circumference of the bar 811 and in use introduce an irregularity into a straight flow and create a vortex. The bar 811 is made of stainless steel and the wires may be integral or fused to the bar 811. The bar is fixed within the channel 610 such that it is not movable.

The second end 510 of the probe 110 is provided with a collar (not shown) extending therefrom and extending around the circumference of the channel 610. The collar is sized to be connected to a connecting means (not shown) to connect the probe 100 to a suction pump (not shown) as described in any one of the above embodiments. The collar is received within the connecting means by means of male female engagement.

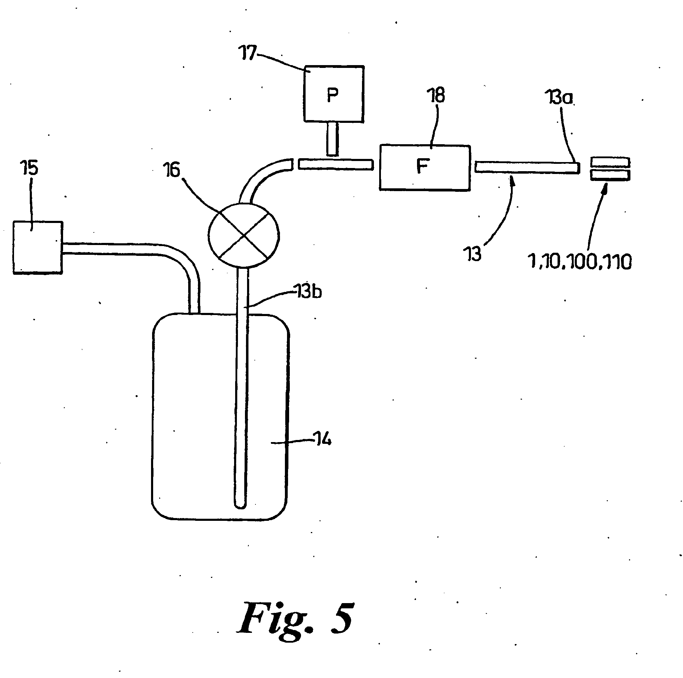

Use of a device comprising the probe 1 as described in any of Figures 1 to 4 is schematically described by reference to Figure 5.

In use the probe 1, 10, 100, 110 is connected to one end of an elongate connection means 13, which is a standard diameter flexible tube, by fitting the collar of the probe into one end 13a of the connection means 13, by male female engagement between the parts. With the probe of Figure 1 the flexible tube 3 may be joined with the connection means 13 or alternatively the flexible tube 3 and the connection means 13 may be one and the same.

The second end 13b of the connection means 13 is secured to a blood collection vessel 14. The vessel has a capacity of 200 - 350ml and is of rigid construction.

Also connected to the blood collection vessel 14 is a vacuum pump 15. The pump is provided with a control means (not shown) to control the level of suction provided by the pump during use. The control means may include a pressure transducer 17.

The connections of the pump 15 and the connection means 13 to the blood collection vessel 14 are sealed to ensure that when the pump is activated it acts on the probe through the collection vessel 14 and the connection means 13 without loss of pressure between the pump 15 and the probe 1.

The device is also provided with means 18 to monitor the amount of blood flowing into the vessel through the probe. The means 18 to monitor the amount of blood flowing into the vessel is a magnetic flow meter that monitors the flow of blood through the connection means 13, as the diameter of the connection means is known it is possible to calculate the amount of blood being removed from the patient. Up to approximately 180ml of blood can be safely removed from the patient.

The device is also provided with a solenoid valve 16 to close the connection means 13 by use of the control means when the blood clot has been removed or when enough blood has been removed.

Once it has been established using an on table angiogram that the patient has undergone a thrombo-embolic stroke and the position of the clot has been determined by angiogram a guidewire with its protective plastic sheathing is inserted into the appropriate artery. The guide wire is positioned 3 to 5mm from the clot. The probe and connection means is inserted over the guide wire into the artery, using standard catheter techniques. The probe position is monitored using X-ray or angiogram and the probe is moved along the patient's blood circulatory system until the clot is reached. The probe is positioned close to but not touching the clot (3 to 5mm from the clot) and primed with saline or any fluid compatible with bodily fluids to prevent air emboli

The vacuum pump is then activated and the pressure transducer sets the pressure that it is to operate at. The control means monitors the pressure from the pump and feeds back to the pump if any adjustment is required to maintain a constant pressure.

The control means can also be set to shut off the pump using the solenoid valve if more than 180ml of blood is collected in the blood collection vessel.

As a result a vortex is created around the first end of the probe. The clot is removed from the blood vessel wall and sucked into the probe where it either remains, held under suction, while the probe is removed; or it passes through the probe into the collection vessel. The monitoring means then notes the capture of a clot and causes the solenoid valve to cut off the pump before the probe is removed from the patient.

The patient is then subjected to usual aftercare procedures for catheter processes and monitored as a stroke patient for any long term damage caused by the stroke.

## Claims

1. A probe (1, 10, 100, 11) adapted for use in retrieving blood clots from part of the blood circulatory system, the probe having a first end (4, 40, 400, 410) and a second (5, 50, 500, 510) end wherein each said end is provided with an aperture therein and the aperture in the second end is adapted in use to be connected to a suction pump (15) by a connection means (13), a channel (6, 60, 600, 610) passing from the aperture in the second end of the probe to the aperture in the first end, wherein the probe has no moving parts and is **characterised by** a spiral element and/or rifling element on all or part of the wall of the channel and/or a bar with any suitable cross-section extending across at least part of the diameter of the channel and/or an elongate bar extending along part of the length of the channel with at least one spiral to give rise, in use, to the creation of a shear force by flow around the first end of the probe.

2. A probe according to claim 1 that is generally cylindrical in configuration.

3. A probe according to any of the preceding claims in which the channel (6) between the second end and first end of the probe is, or has a portion, of increasing diameter as it extends from the second end to the first end.

4. A probe according to claim 3 in which the spiral element and or rifling element is provided on the surface of the wall of the portion of the channel of increased diameter.

5. A probe according to claim 3 or claim 4 in which the diameter increases such that the wall of the channel is at an angle of 20 to 40° to the longitudinal axis of the channel.

6. A probe according to any of the preceding claims in which the spiral element and or rifling element is positioned at an angle of 20 to 35° to the longitudinal axis of the probe.

7. A probe according to any of the preceding claims wherein the elongate bar (810) extends along the channel (610) and is provided with one or more spirals of wire (811 a, 811 b, 811 c) secured around at least part of its length.

8. A probe according to any of the preceding claims wherein the elongate bar extending along the channel is a screw-threaded bar.

9. A probe according to any of claims 7 to 8 in which the elongate bar (810) is secured in the channel of the probe so that a flow path is created around the bar thus leading to the creation of a twisting shear force.

10. A probe according to any preceding claim in which the bar has a length of at least 40% of the diameter of the aperture and extends from one part of the edge of the aperture to another part of the edge of the aperture.

11. A probe according to any one of claims 1 to 6, wherein the probe comprises one or more protrusions, or fins, extending from the outer surface of the probe.

12. A probe according to any of the preceding claims that is releasably connected to a suction pump (15) by means of said connection means (13).

13. A probe according to any of the preceding claims manufactured from polypropylene, poly ether-poly amide co-polymer / polyether block amide (PEBAX) or Teflon.

14. A probe according to any one of claims 1 to 12 manufactured from stainless steel, titanium, aluminium, anodised aluminium or a ceramic.

15. A probe according to any of the preceding claims in which the thickness of the wall of the channel of the probe is up to 1 mm.

16. A probe according to claim 1 wherein the bar comprises a V shaped and/or square and/or semi-circular and/or triangular cross-section and/or screwthread.

## Patentansprüche

1. eine Sonde (1, 10, 100, 11) für den Einsatz im Abrufen von Blutgerinnseln aus Teil des Blutes angepasst Herz-Kreislauf-System, die Sonde mit einem ersten End (4, 40, 400, 410) und kurz (5, 50, 500, 510) Ende, wobei jeder Ende sagte, ist mit einer Blende darin und die Blende in das zweite Ende ist geeignet im Einsatz mit einer Saugpumpe (15) mit einem Anschluss (13), verbunden sein ein Kanal (6, 60, 600, 610) Übergabe von der Blende in das zweite Ende der Sonde an die Blende im ersten Ende, wobei die Sonde hat keine beweglichen Teile und zeichnet sich durch eine Spirale Element und/oder Drall Element auf alle oder Teil der Wand des Kanals und/oder eine Bar mit einer geeigneten Querschnitt Ausdehnung auf zumindest einen Teil des Durchmessers der Kanal bzw. ein verlängernd bar entlang Teil der Länge des Kanals mit mindestens einer Spirale führen, im Einsatz, zur Schaffung einer Scherung zu erzwingen, indem Sie Luftstrom um das erste Ende der Sonde.

2. eine Sonde nach Anspruch 1, die in der Konfiguration in der Regel zylindrisch ist.

3. eine Sonde nach einem der vorhergehenden Ansprüche, in dem der Kanal (6) zwischen den zweite Ende und erste Ende der Sonde ist, oder einen Teil des zunehmenden Durchmesser hat, wie es vom zweiten Ende bis zum ersten Ende erstreckt.

4. eine Sonde nach Anspruch 3, in dem die Spirale-Element und oder Drall Element ist, zur Verfügung gestellt, auf der Oberfläche der Mauer des Teils des Kanals der erhöhte Durchmesser.

5. eine Sonde nach Anspruch 3 oder 4, in dem der Durchmesser, so erhöht, dass die Wand des Kanals ist in einem Winkel von 20 bis 40 Grad zur Längsachse des Kanals.

6. eine Sonde nach einem der vorhergehenden Ansprüche, in dem die Spirale-Element und oder Drall Element befindet sich in einem Winkel von 20 bis 35 Grad zur Längsachse der Sonde.

7. eine Sonde nach einem der vorhergehenden Ansprüche, worin die verlängernd bar (810) erstreckt sich entlang des Kanals (610) und verfügt über eine oder mehrere Spiralen Draht (811a, 811b, 811c) gesichert, um zumindest einen Teil seiner Länge.

8. eine Sonde nach einem der vorhergehenden Ansprüche, worin die verlängernd bar erweitern entlang des Kanals ist eine Schraube Singlethreaded-Bar.

9. eine Sonde nach einem der Ansprüche 7 bis 8, in dem die verlängernd bar (810) gesichert ist der Kanal der Sonde so dass ein Flusspfad rund um die Bar entsteht führt zur Entstehung einer Verdrehung Schubkraft.

10. eine Sonde nach vorhergehenden Ansprüche, in denen die Bar hat eine Länge von mindestens 40 % der der Durchmesser der Blende und erstreckt sich von einem Teil der Kante der Öffnung in einen anderen Teil des Randes der Blende.

11. eine Sonde nach einem der Ansprüche 1 bis 6, wobei die Sonde mindestens eine vorstehende Teile, oder flossen umfasst, ausgehend von der äußeren Oberfläche der Sonde.

12. eine Sonde nach einem der vorhergehenden Ansprüche, die an eine Saugpumpe (15) durch lösbar verbunden ist, sagte Verbindung (13) bedeutet.

13. eine Sonde nach einem der vorhergehenden Ansprüche gefertigt aus Polypropylen, Poly Ether-Poly Amid Co-Polymer / Polyether block Amide (PEBAX) oder Teflon.

14. eine Sonde nach einem der Ansprüche 1 bis 12 gefertigt aus Edelstahl, Titan, Aluminium, eloxiertes Aluminium oder eine Keramik.

15. eine Sonde nach einem der vorhergehenden Ansprüche, in denen die Dicke der Wand des Kanals der Sonde bis zu 1 mm ist.

16. eine Sonde nach Anspruch 1, wobei die Bar einen V geformte und/oder quadratische umfasst, und/oder halbrunden bzw. dreieckigen Querschnitt und/oder Filtrieraufsatz.

## Revendications

1. une sonde (1, 10, 100, 11) conçue pour être utilisés lors de l'extraction des caillots de sang d'une partie du sang système circulatoire, la sonde ayant une fin de première (4, 40, 400, 410) et une seconde (5, 50, 500, 510) fin dans lequel chacun a dit fin est fourni avec une ouverture qui y sont et l'ouverture à la fin du deuxième est adapté en usage à être raccordés à une pompe aspirante (15) par un moyen de connexion (13), un passage de canal (6, 60, 600, 610) depuis l'ouverture de la seconde extrémité de la sonde à l'ouverture à la fin de la première, dans laquelle la sonde n'a aucun déplacement des pièces et se **caractérise par** un élément en spirale et/ou élément de rayures sur l'ensemble ou une partie de la paroi de la chaîne et/ou un bar avec n'importe quel échantillon approprié s'étendant sur au moins une partie du diamètre de la chaîne et/ou un allongé bar s'étendant le long de la partie de la longueur du canal avec au moins une spirale pour donner naissance, l'utilisation, à la création d'un cisaillement forcer en écoulement autour de la première extrémité de la sonde.

2. une sonde selon la revendication 1 qui est généralement cylindrique dans la configuration.

3. une sonde selon tout ce qui précède prétend dans lequel le canal (6) entre le fin de deuxième et de la première extrémité de la sonde est, ou a une partie, de diamètre croissant car elle s'étend de la fin du deuxième à la fin de la première.

4. une sonde selon la revendication 3, dans lequel l'élément de la spirale et ou de rayures élément est fournie sur la surface de la paroi de la partie du chenal du diamètre augmenté.

5. une sonde selon la revendication 3 ou la revendication 4 dans lequel le diamètre augmente tel que le paroi du canal est à un angle de 20 à 40° à l'axe longitudinal du canal.

6. une sonde selon tout ce qui précède prétend dans lequel l'élément de la spirale et ou fusil élément est positionné à un angle de 20 à 35° à l'axe longitudinal de la sonde.

7. une sonde selon tout ce qui précède prétend dans lequel les allongés bar (810) s'étend le long du canal (610) et est fourni avec une ou plusieurs spirales de fil (811 a, 811b, 811c) fixés autour d'au moins une partie de sa longueur.

8. une sonde selon tout ce qui précède prétend dans laquelle le bar extension allongé le long du canal est un bar à bouchon fileté.

9. une sonde selon une quelconque des revendications 7 à 8 dans lequel l'allongés bar (810) sont sécurisé dans le canal de la sonde afin qu'un trajet d'écoulement est créé autour du bar conduisant ainsi à la création d'une force de torsion cisaillement.

10. une sonde selon n'importe quel des revendications précédentes où le bar a une longueur d'au moins 40 % du diamètre de l'ouverture et s'étend d'une partie du bord de l'ouverture à une autre partie du bord de l'ouverture.

11. une sonde selon l'une quelconque des revendications 1 à 6, où la sonde comprend un ou plusieurs saillies ou les nageoires, qui s'étend de la surface externe de la sonde.

12. une sonde selon aucune des revendications précédentes LIBERABLE relié à une pompe aspirante (15) par le biais de dit connexion signifie (13).

13. une sonde selon tout ce qui précède prétend fabriqués de polypropylène, poly éther-poly amide co-polymère / polyéther bloc amide (PEBAX) ou téflon.

14. une sonde selon l'une quelconque des revendications 1 à 12 fabriqués à partir d'acier inoxydable, titane, aluminium, anodisé en aluminium ou en céramique.

15. une sonde selon aucune des revendications précédentes où l'épaisseur de la paroi du canal de la sonde est jusqu'à 1 mm.

16. une sonde selon la revendication 1, où le bar comprend un V en forme et/ou carrée ou section semi-circulaire ou triangulaire ou vis.
